# FASCICULE DE BREVET EUROPEEN

(11) **EP 2 544 312 B1**
(45) Date de publication et mention de la délivrance du brevet: **11.02.2015**
(21) Numéro de dépôt: 12172600.4
(22) Date de dépôt: 19.06.2012
(51) Int. Cl.: H01R 13/514, H01R 24/58, H01R 13/187, H01R 13/52, A61N 1/08

(54) **Connecteur pour sonde multipolaire**
Anschluss für multipolare Sonde
Connector for multipolar probe

(30) Priorité: 06.07.2011 FR 1156099
(43) Date de publication de la demande: 09.01.2013
(73) Titulaire: Sorin CRM SAS, 92140 Clamart Cedex (FR)
(72) Inventeur: Lucas, François, 92120 MONTROUGE (FR); Jullien, Elodie, 69570 DARDILLY (FR)
(74) Mandataire: Dupuis-Latour, Dominique

(56) Documents cités:
- EP-A1- 1 641 084
- WO-A1-2011/017432
- DE-A1- 10 339 958
- US-A- 5 730 628
- US-A1- 2003 073 348
- US-A1- 2005 186 829
- US-A1- 2007 202 728
- US-A1- 2008 274 651
- US-A1- 2011 059 639
- US-B1- 7 083 474

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boîtier généralement désigné "générateur", raccordé électriquement et mécaniquement à une ou plusieurs "sondes" munie(s) d'électrodes destinées à venir en contact avec les tissus sur lesquels on souhaite appliquer des impulsions de stimulation et/ou recueillir un signal électrique : myocarde, nerf, muscle, ...

II existe des systèmes de connexion normalisés permettant de garantir l'interchangeabilité des sondes et des générateurs produits par différents fabricants.

Ainsi, les standard dits "IS-1" et "IS-4" définissent un certain nombre de caractéristiques dimensionnelles et électriques relatives aux sondes de délivrance d'impulsions de stimulation. Pour les sondes de défibrillation ou de cardioversion, où les contraintes électriques sont plus sévères compte tenu de l'énergie élevée devant transiter du générateur à la sonde, les standards dits "DF-1" et "DF-4" définissent les caractéristiques dimensionnelles et électriques du système de connexion.

La complexité de ces sondes, qui intègrent déjà les contraintes spécifiques en termes d'énergie électrique liées à l'une ou l'autre des utilisations, stimulation ou choc, est encore accrue par le développement des dispositifs multisite et des capteurs intracardiaques, tels que les capteurs d'accélération endocavitaire (EA). Du point de vue de la connectique, ceci conduit à une multiplication des fiches de connexion, avec au surplus des standards différents selon les fiches.

On comprend l'avantage que peut représenter une fiche unique, soumise à un standard unique, portant une pluralité de contacts électriques permettant d'assurer simultanément l'établissement des connexions aux diverses bornes du générateur pour tous les niveaux d'énergie, qu'il s'agisse du recueil de signaux de dépolarisation, de l'application d'impulsions de stimulation ou de l'envoi d'une énergie de choc de défibrillation.

Dans ce cadre, il a été proposé une fiche de connexion unique du type "isodiamètre", c'est-à-dire de forme cylindrique uniforme, destinée à être insérée dans une cavité cylindrique homologue de la tête de connecteur du générateur.

Le EP 1 641 084 A1 (ELA Medical) décrit une telle fiche de connexion isodiamètre dont la surface cylindrique extérieure présente un empilement de zones de contact électrique annulaires, réalisées par des bagues cylindriques conductrices, et de zones cylindriques isolantes destinées à isoler électriquement les bagues conductrices.

Pour recevoir une fiche de connexion multipolaire de ce type et la connecter aux circuits électriques du générateur, il est prévu des connecteurs comprenant une cavité constituée de manière similaire à la fiche elle-même, au sens où elle comporte également un empilement d'éléments annulaires de contact électrique aptes à réaliser un contact électrique avec les bagues conductrices correspondantes de la fiche de connexion. Dans cet empilement, les éléments annulaires de contact électrique sont séparés alternativement par des éléments annulaires d'isolement.

On remarquera que, contrairement aux réalisations précédentes (standard IS1/DF1) où les éléments d'isolement sont portés par la sonde, ces derniers sont maintenant disposés dans la cavité (standard IS4/DF4), ce qui présente l'avantage de fournir des éléments d'isolement neufs chaque fois que le générateur est remplacé.

Le terme d"'isolement" employé ici concerne aussi bien i) l'étanchéité de la cavité par rapport au milieu extérieur, vis-à-vis en particulier des liquides corporels, que ii) l'isolation électrique, c'est-à-dire l'isolation basse tension et haute tension des éléments de contact électrique de la cavité, ainsi que des éléments de contact électrique de la cavité avec le milieu extérieur.

Le US 2005/0186829 A1 décrit un connecteur comprenant une série de pièces empilables, encliquetables entre elles avec interposition alternativement d'un joint souple ou d'un organe de connexion électrique. Chaque joint souple, ou organe de connexion, est ainsi pincé entre deux de ces pièces homologues encliquetables. L'étanchéité de ce connecteur n'est toutefois pas assurée de façon satisfaisante à l'interface entre ces deux pièces encliquetables, ce qui laisse subsister le risque d'infiltrations depuis l'environnement vers l'intérieur du connecteur, notamment au niveau des organes de connexion électrique. II est de ce fait nécessaire de prévoir un niveau d'étanchéité supplémentaire, par exemple par collage des pièces encliquetables, montage à force de type "press-fit" ou autre moyen analogue.

Le US 7 083 474 B1 propose également un joint souple surmoulé sur un noyau de rigidification portant des trous traversants. Le noyau rigide réalise une butée lors de l'empilement des différents éléments de la cavité. Toutefois le joint n'étant pas ancré mais simplement attaché au noyau rigide par les trous traversants, rien ne l'empêche de pouvoir se déplacer au cours de l'insertion de la fiche de connexion, ce qui risque de dégrader l'étanchéité et l'isolation électrique.

Le US 2003/0073348 A1 décrit un autre connecteur encore, réalisé à partir d'un empilement d'éléments porteurs alternativement de joints et d'organes de connexion électrique. Mais les inconvénients décrits plus haut (étanchéité imparfaite et nécessaire recours à un niveau d'étanchéité supplémentaire, par exemple par collage ou "press-fit") demeurent entiers.

Aussi, un but de l'invention est de proposer un connecteur qui garantisse non seulement un positionnement précis des éléments de contact électrique et d'isolement entre eux, mais également une étanchéité et une isolation électrique parfaites en toutes circonstances, en particulier lorsque la fiche de connexion est engagée dans la cavité du connecteur.

Ce but est atteint, conformément à l'invention, grâce à un connecteur pour un générateur de dispositif médical implantable actif. Ce connecteur comprend une cavité sensiblement cylindrique, destinée à recevoir une sonde multipolaire, comportant, comme divulgué par le US 2005/0186829 A1 précité, un empilement d'éléments annulaires de contact électrique séparés alternativement par des éléments annulaires d'isolement. Les éléments d'isolement comprennent un manchon rigide annulaire et un joint souple annulaire disposé, dans une région intérieure du manchon, contre une face annulaire interne du manchon. Le manchon et le joint comprennent des moyens respectifs d'immobilisation du joint par rapport au manchon en direction axiale, au moyen de profils conjugués définis respectivement sur une face annulaire interne du manchon et sur une face annulaire externe du joint. De façon caractéristique de l'invention, le joint s'étend axialement d'une face latérale du manchon jusqu'à l'autre dans ladite région intérieure du manchon et chacune des faces latérales du joint souple est en saillie par rapport à la face latérale correspondante du manchon.

Ici et dans la suite, on qualifiera de "souple" un matériau présentant une dureté inférieure ou égale à 80 Shore A, et par "dur" un matériau présentant une dureté supérieure ou égale à 70 Shore D.

Ainsi, les éléments d'isolement du connecteur selon l'invention se présentent en deux parties, à savoir un manchon rigide de forme générale annulaire et un joint souple, également de forme générale annulaire, destiné à venir en contact avec une zone cylindrique isolante de la fiche de connexion de manière à assurer l'étanchéité de la cavité et l'isolation électrique basse tension entre deux bagues conductrices consécutives de la fiche.

Le joint souple est disposé à l'intérieur du manchon rigide, sa face annulaire externe étant appliquée contre la face annulaire interne du manchon. En outre, le joint souple est maintenu sur le manchon rigide en position axiale, c'est-à-dire dans la direction de l'axe de la cavité cylindrique, par les moyens d'immobilisation définis sur le joint et sur le manchon.

De cette manière, le joint ne peut en aucun cas se déplacer, même lorsqu'il est soumis à des contraintes axiales lors de l'insertion de la fiche de connexion dans la cavité.

De plus, la saillie de chacune des faces latérales du joint souple par rapport à la face latérale correspondante du manchon rigide permet de créer par compression axiale du joint une étanchéité entre la cavité et le milieu extérieur et de supprimer les courants de fuite entre le joint et le manchon.

L'invention prévoit plus spécialement que les profils conjugués des moyens d'immobilisation sont définis par des épaulements ménagés en coopération sur la face annulaire interne du manchon et sur la face annulaire externe du joint, ou que les profils conjugués comportent des gorges et des nervures ménagées en coopération sur la face annulaire externe du joint et sur la face annulaire interne du manchon.

Le manchon peut être constitué par une pièce unique ou en deux demi-manchons munis d'ergots et de fraisages de clippage des demi-manchons entre eux.

Selon un autre aspect de l'invention, les éléments de contact électrique comprennent une cage logeant un contact élastique, le contact élastique comprenant une pièce métallique élastique présentant une section enroulée en forme de U pour constituer un cylindre ouvert, apte à réaliser une fonction de contact souple avec des bagues conductrices homologues de la fiche de connexion de la sonde. Le contact élastique peut notamment être réalisé en une seule pièce à partir d'une feuille plate de métal élastique découpée de manière à y former des lamelles, et pliée afin d'obtenir la forme en U, puis le cylindre ouvert. Avantageusement, la cage comprend deux parois latérales formant butées axiales de positionnement pour les éléments annulaires d'isolement adjacents de l'empilement.

Le connecteur est constitué par surmoulage d'un empilement d'éléments de contact électrique et d'éléments d'isolement. Dans ce cas, les zones de fusion entre les matériaux constituant les manchons et le connecteur réalisent l'étanchéité entre les éléments de contact électrique et entre la cavité et le milieu extérieur.

Enfin, l'alignement et la coaxialité des différents éléments de la cavité sont garantis du fait que les éléments de contact électrique et les manchons des éléments d'isolement comprennent des moyens d'assemblage par emboîtement.

Selon un autre mode de réalisation, le connecteur est constitué, selon l'invention, par insertion des éléments de contact électrique et des éléments d'isolement à l'intérieur de la cavité. Les éléments d'isolement sont alors constitués d'un deuxième joint souple annulaire disposé sur la face annulaire externe du manchon pour assurer l'isolation entre les éléments de contact électrique et entre la cavité et le milieu extérieur.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1a est une vue en perspective d'un premier mode de réalisation d'une tête de connecteur conforme à l'invention.
La Figure 1b est une vue en coupe de la tête de connecteur de la Figure 1a.
La Figure 2a est une vue en perspective d'un élément de contact électrique de la tête de connecteur des Figures 1 a et 1 b.
La Figure 2b est une vue en perspective éclatée de l'élément de contact électrique de la Figure 2a.
La Figure 3a est une vue en perspective d'un élément d'isolement de la tête de connecteur des Figures 1 a et 1 b.
La Figure 3b est une vue en perspective éclatée de l'élément d'isolement de la Figure 3a.
La Figure 4a est une vue en perspective éclatée d'une première variante de réalisation de l'élément d'isolement des Figures 3a et 3b.
La Figure 4b est une vue en coupe de l'élément d'isolement de la Figure 4a.
La Figure 5a est une vue en perspective éclatée d'une deuxième variante de réalisation de l'élément d'isolement des Figures 3a et 3b.
La Figure 5b est une vue en coupe de l'élément d'isolement de la Figure 5a.
La Figure 6a est une vue en perspective d'un empilement d'éléments de contact électrique des Figures 2a et 2b et d'éléments d'isolement des Figures 3a et 3b.
La Figure 6b est une vue en perspective éclatée de l'empilement de la Figure 6a.
La Figure 7 est une vue en coupe d'un deuxième mode de réalisation d'une tête de connecteur conforme à l'invention.
La Figure 8 est une vue en perspective éclatée d'un empilement d'éléments de contact électrique et d'éléments d'isolement de la tête de connecteur de la Figure 7.
La Figure 9a est une vue en perspective éclatée d'un l'élément d'isolement de la tête de connecteur de la Figure 7.
La Figure 9b est une vue en coupe de l'élément d'isolement de la Figure 9a.

On va maintenant décrire divers exemples de réalisation du dispositif de l'invention.

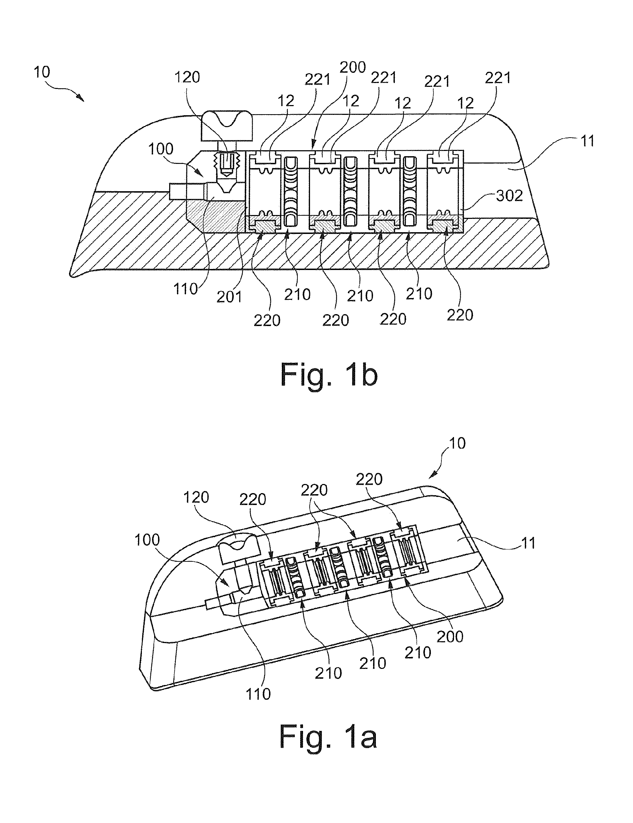

Sur les Figures 1a et 1b est représentée une tête 10 de connecteur pour un générateur de dispositif médical implantable actif.

La tête 10 de connecteur comprend une cavité 11 sensiblement cylindrique destinée à recevoir, en conformité avec la norme IS-4/DF-4 (ISO 27186-2010), une fiche de connexion d'une sonde multipolaire (non représentée), de manière à pouvoir échanger des signaux électriques entre le générateur du dispositif et le coeur d'un patient.

Le EP 1 641 084 A1 précité décrit une telle fiche de connexion à quatre pôles. Plus précisément, cette fiche de connexion connue est du type isodiamètre présentant à une de ses extrémités une broche de contact électrique axiale et, sur le corps même de la fiche, trois zones de contact électrique annulaires réalisées par des bagues cylindriques conductrices. Les zones de contact électrique annulaires sont séparées alternativement par des zones cylindriques isolantes intercalaires afin d'isoler électriquement les zones de contact électrique les unes des autres. On peut ainsi, en enfichant d'un seul geste la fiche de connexion dans la cavité 11 de la tête 10, réaliser simultanément toutes les liaisons électriques nécessaires entre le générateur et les pôles de la sonde. A cet effet, la cavité 11 doit, bien entendu, être munie d'éléments homologues à ceux présents sur la fiche de connexion. Ces éléments vont maintenant être décrits en détail en relation avec la norme précitée.

Comme le montrent les Figures 1 a et 1 b, la cavité 11 de la tête 10 comprend un insert 100 à vis permettant, d'une part, d'introduire la broche axiale de la fiche de connexion dans une douille 110 afin de réaliser la liaison électrique de la broche avec le générateur, et de fixer la fiche dans la cavité 11 au moyen de la vis 120.

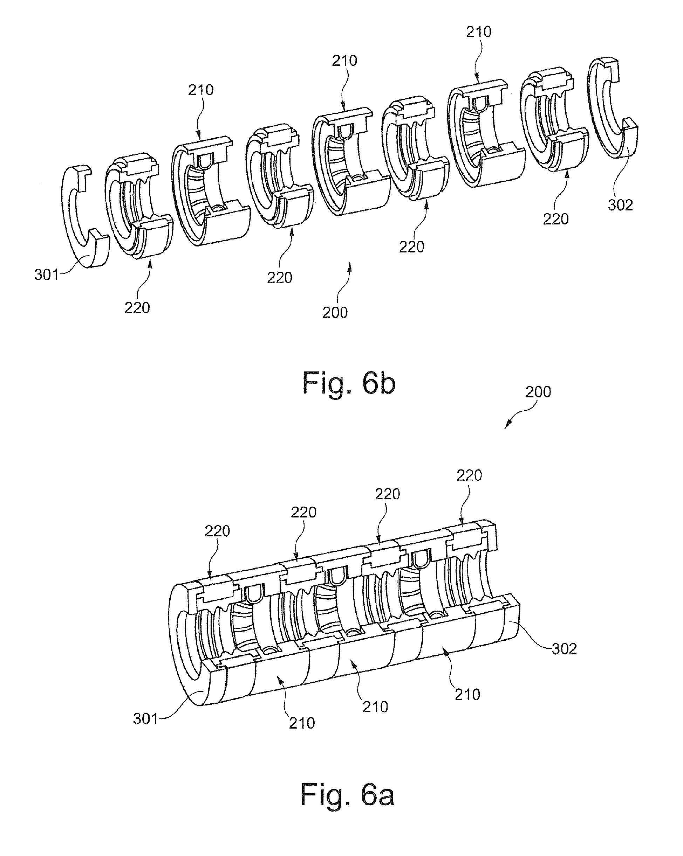

La cavité 11 contient en outre un empilement 200 d'éléments de contact électrique et d'isolement que l'on peut voir également sur les Figures 6a et 6b.

Plus précisément, l'empilement 200 comprend trois éléments 210 de contact électrique annulaires prévus pour réaliser un contact électrique avec les bagues conductrices correspondantes de la fiche de connexion dans le but de transmettre, avec la broche axiale, l'ensemble des signaux électriques haute et/ou basse tension de l'électronique du générateur vers le coeur via la sonde multipolaire implantée et inversement. Ces éléments de contact électrique 210 doivent pouvoir recevoir les fiches de connexion des sondes et répondre aux exigences d'effort d'insertion et d'extraction et de performances électriques définies dans la norme.

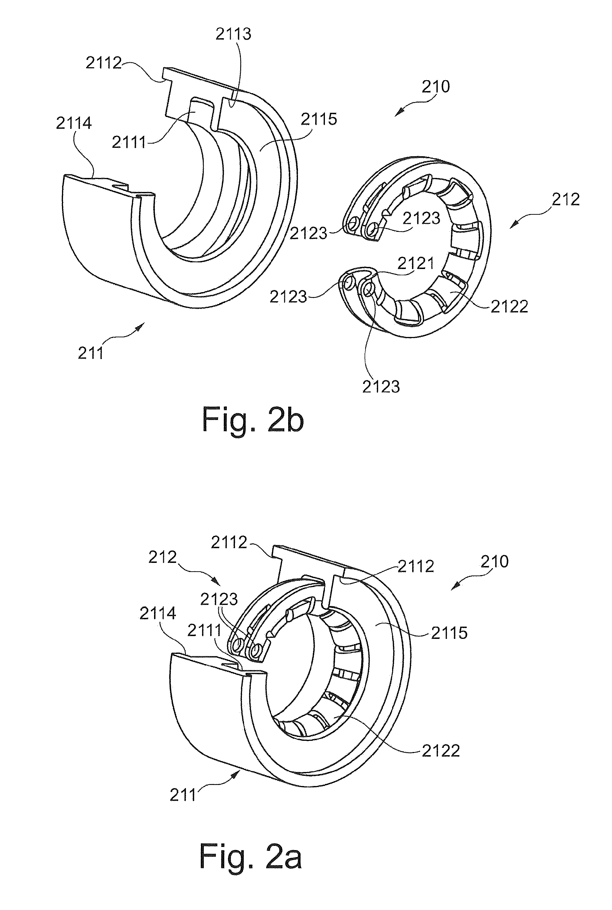

On peut voir avec plus de détails sur les Figures 2a et 2b que les éléments 210 de contact électrique, ou simplement "contacts électriques", sont composés de deux sous-éléments cylindriques annulaires, à savoir une cage 211, en matériau biocompatible tel que le MP35N ou l'acier inox, et un contact élastique 212 reçu par la cage 211 à l'intérieur d'une gorge 2111 ménagée dans sa partie interne, le long de son diamètre intérieur. De plus, la cage 211 comporte des épaulements externes 2112, 2113 permettant, comme on le verra plus loin, d'assurer l'alignement et la coaxialité des différents éléments de la cavité.

Le contact élastique 212 se compose d'une seule pièce en métal élastique implantable présentant une section 2121 enroulée en forme de U pour constituer un cylindre ouvert. Plusieurs lamelles 2122 de largeur identique sont réparties uniformément sur le cylindre ouvert. Enfin, quatre trous 2123 sont percés aux extrémités du cylindre ouvert.

Le contact élastique 212 réalise une fonction de contact souple avec les bagues conductrices de la fiche de connexion de la sonde de façon à obtenir le meilleur contact électrique et de faibles résistances de contact. Sa section en U 2121 permet d'éviter les arêtes vives pouvant dégrader les éléments directement en contact et facilite la mise en forme du contact élastique. Les trous 2123, ou tous autres moyens équivalents, acceptent un outil pour les différentes manipulations au cours de la fabrication et de la mise en place du contact élastique 212 dans la cage 211. La précharge exercée sur les trous 2123 diminue le diamètre du contact élastique 212 permettant sa mise en place dans la gorge 2111. Après suppression de la précharge, le contact élastique 212 se détend et reste maintenu dans la gorge 2111.

Les deux parois latérales 2114, 2115 de la cage 211 servent de butées axiales de positionnement pour les différents éléments de la cavité au moment de leur empilement. Son diamètre extérieur est une zone de connexion électrique des fils reliant les contacts élastiques 212 à l'électronique du générateur, par soudure laser ou électrique.

La réalisation du contact élastique 212 se fait à partir d'une feuille plate de métal élastique implantable. La découpe des formes des lamelles 2122 est effectuée à plat par découpe chimique, découpe au jet d'eau ou tout autre procédé industriel. Des pliages sont ensuite réalisés afin d'obtenir la forme de U 2121, puis le cylindre ouvert. La découpe des lamelles 2122 peut aussi être obtenue après les opérations de pliage.

On peut observer avantageusement que le contact électrique a un encombrement réduit qui favorise la conception du reste de la cavité et de la fiche de connexion de la sonde. La forme en U 2121 du contact élastique 212 assure une bonne conductivité électrique car les surfaces en appui entre le contact élastique 212 et la gorge 2111 garantissent une distribution des appuis uniforme. Le contact élastique 212 est réalisé à partir d'une seule pièce découpée et pliée. Aucune opération, de soudure notamment, n'est plus nécessaire.

Bien entendu, le nombre de lamelles 2122 et/ou l'épaisseur de la feuille plate peuvent être augmentés ou diminués de manière à modifier l'élasticité du contact et/ou la résistance de contact afin d'optimiser les performances du contact électrique 210.

Conformément aux Figures 1a et 1b, les contacts électriques 210 sont séparés alternativement le long de l'empilement 200 par des éléments annulaires 220 d'isolement disposés en regard des zones isolantes de la fiche de connexion de manière à créer une isolation électrique entre les contacts électriques 210 entre eux, et réaliser l'étanchéité de la cavité 11 vis-à-vis du milieu extérieur. Les éléments 220 d'isolement doivent de plus garantir une isolation haute tension et supprimer les chemins de fuite au niveau de leur diamètre intérieur, à l'interface avec les zones isolantes de la fiche de connexion, ainsi qu'au niveau de leur diamètre extérieur, à l'interface avec la cavité 11.

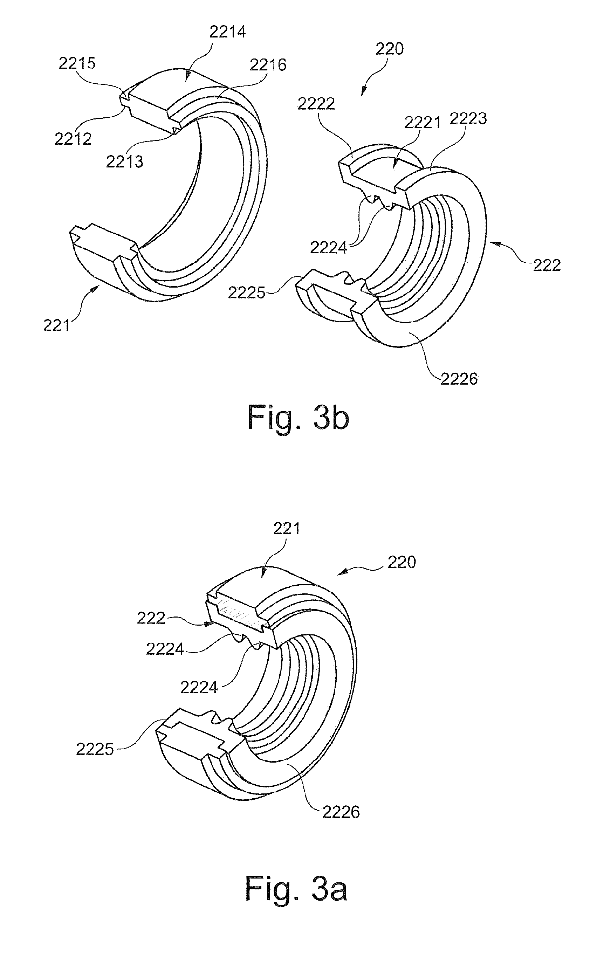

Comme le montrent les Figures 3a et 3b, les éléments 220 d'isolement se composent de deux sous-éléments cylindriques, à savoir un manchon 221 en thermoplastique rigide, comme un polyuréthanne de grade dur, notamment le Tecothane (marque déposée), à l'intérieur duquel est disposé un joint souple 222 en élastomère tel que le silicone ou un polyuréthanne de grade souple. La face annulaire interne du joint souple 222 est munie d'anneaux 2224 d'étanchéité capables de se comprimer radialement sur une zone isolante de la fiche de connexion lorsque cette dernière est insérée dans la cavité 11, assurant ainsi l'isolation entre deux bagues conductrices consécutives de la fiche et les contacts élastiques 212 correspondants.

Le joint souple 222 est placé à l'intérieur du manchon rigide 221 contre une face annulaire interne 2211 et y est maintenu grâce à des moyens d'immobilisation qui, d'une façon générale, sont constitués par des profils conjugués définis respectivement sur la face annulaire interne 2211 du manchon 221 et sur la face annulaire externe 2221 du joint 222.

Dans l'exemple de réalisation des Figures 3a et 3b, les profils conjugués sont définis par des épaulements internes 2212, 2213 ménagés sur la face annulaire interne 2211 du manchon 221 de manière à coopérer avec des épaulements externes 2222, 2223 ménagés sur la face annulaire externe 2221 du joint 222.

On garantit ainsi que le joint souple 222 reste bien en position en particulier lorsque la fiche de connexion est introduite dans la cavité 11.

Les épaulements internes 2212, 2213 du manchon 221 permettent de créer une barrière au courant de fuite au niveau de l'interface entre le manchon 221 et le joint souple 222, tandis que les épaulements externes 2215, 2216 ménagés sur la face externe 2214 du manchon 221 assurent l'alignement et la coaxialité des différents éléments lors de leur assemblage en s'emboîtant avec les épaulements externes 2112, 2113 de la cage 211 du contact électrique 210.

Ainsi qu'on peut le voir plus particulièrement sur la Figure 3a, les faces latérales 2225, 2226 du joint souple 222 sont en saillie par rapport aux faces latérales correspondantes du manchon 221, ce qui permet, par compression axiale, de créer une étanchéité et de supprimer les courants de fuite entre le joint souple 222 et le manchon 221.

Le joint souple 222 peut-être surmoulé ou assemblé dans la partie interne du manchon 221. Le manchon 221 est moulé ou usiné.

Les avantages du mode de réalisation de l'élément 220 d'isolement présenté aux Figures 3a et 3b sont multiples. II permet en effet de concilier deux facteurs, l'élasticité nécessaire de la forme des anneaux d'étanchéité 2224 et la rigidité imposée à la forme extérieure du manchon 221. De plus, l'association des deux pièces, manchon et joint, garantit le positionnement axial de façon à respecter les zones isolantes ainsi que le positionnement radial permettant l'isolation au niveau du diamètre intérieur du joint 222, conformément aux exigences du standard DF4.

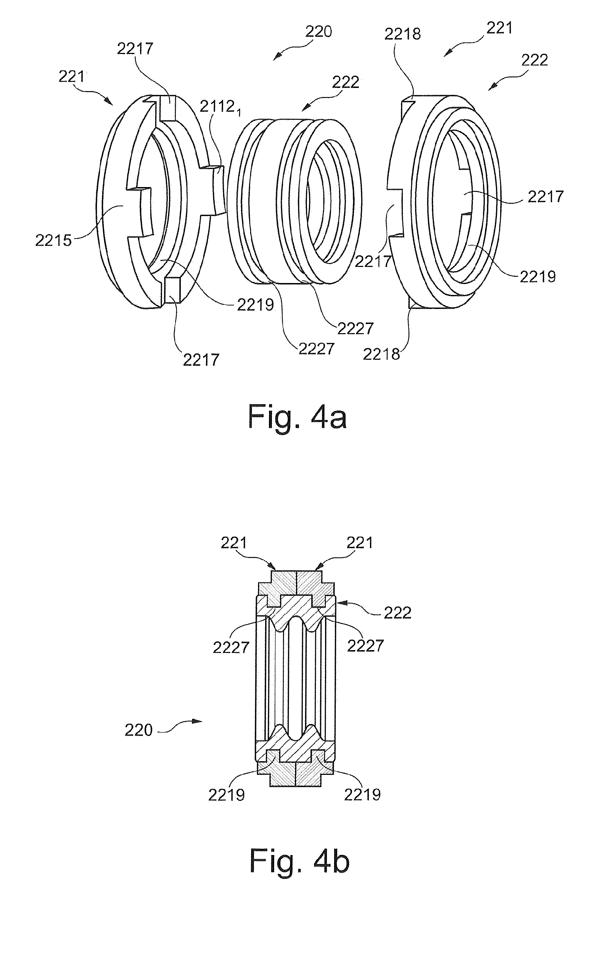

Les Figures 4a, 4b, d'une part, et 5a, 5b, d'autre part, illustrent deux variantes de réalisation mettant en oeuvre un élément d'isolement 220 constitué de deux demi-manchons rigides et d'un joint souple.

Le manchon des Figures 4a et 4b se compose de deux demi-manchons cylindriques identiques 221 assemblés par clippage au moyen de fraisages 2217 et d'ergots 2218 formés sur les faces latérales internes des demi-manchons.

Les profils conjugués d'immobilisation du joint souple 222 comprennent, outre les épaulements décrits précédemment en regard des Figures 3a et 3b, des gorges 2227 portées par la face annulaire externe du joint souple 222 et destinées à coopérer avec des nervures correspondantes 2219 ménagées sur les faces annulaires internes des demi-manchons 221.

Si cela est nécessaire pour augmenter les performances d'isolation, les faces latérales du joint souple 222 peuvent également déborder des faces latérales externes des demi-manchons 221.

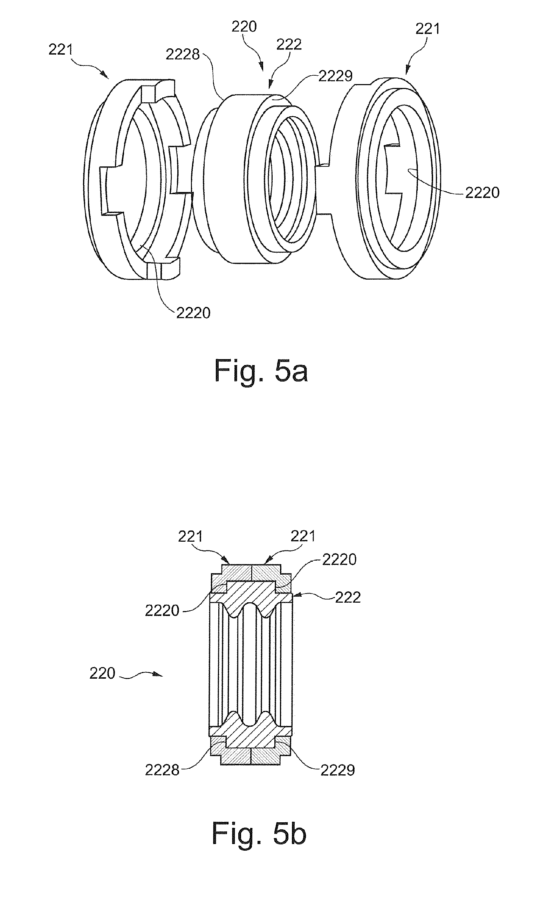

De même, le manchon des Figures 5a et 5b se compose de deux demi-manchons cylindriques identiques 221 présentant des épaulements internes 2220. Comme pour l'élément 220 d'isolement des Figures 4a et 4b, des ergots et des fraisages servent à clipper les deux demi-manchons 221 entre eux. Le joint souple 222 se compose d'une pièce comprenant deux épaulements externes 2228, 2229. Lors de l'assemblage des deux demi-manchons 221 et du joint souple 222, les épaulements externes 2228, 2229 du joint viennent se positionner contre les épaulements internes 2220 des demi-manchons, lesquels exercent une compression sur le joint au niveau des épaulements externes.

Pour former la tête 10 de connecteur des Figures 1a et 1b, les éléments 100, 210, 220 préalablement assemblés mécaniquement sont surmoulés au moyen d'un matériau thermoplastique comme le Tecothane. Ce procédé permet de positionner avec précision les différents éléments et de les maintenir fermement en position. II permet également d'assurer l'isolation entre deux contacts électriques 210 et entre les contacts électriques et le milieu environnant puisqu'une liaison chimique se crée entre les manchons 221 et la tête 10 en Tecothane, dans des zones 12 appelées zones de fusion indiquées sur la Figure 1 b.

Le manchon rigide 221 permet de protéger le joint souple 222 des pression et température d'injection pendant le surmoulage ou le moulage à froid (*casting*).

En pratique, les trois contacts électriques annulaires 210 et les quatre éléments 220 d'isolement sont assemblés alternativement par emboîtement sur une broche de moule de façon à les aligner et constituer un empilement 200, tel que représenté sur les Figures 6a et 6b. Les éléments d'isolement d'extrémité sont encadrés par des bouchons 301, 302 de manière à pouvoir appliquer sur les éléments de l'empilement 200 une pression contrôlée, nécessaire et suffisante pour permettre la mise en butée mécanique de chacun des éléments. Sous l'action de cette pression, les faces latérales 2225, 2226 des joints souples 222 sont comprimées axialement, ce qui crée une barrière au chemin de fuite et de claquage électrique. Cette compression axiale évite également que le matériau thermoplastique de la tête 10 ne s'infiltre à l'intérieur de la cavité 11 pendant l'opération de surmoulage. La broche ainsi équipée de l'ensemble des éléments est placée dans le moule avec la douille 110 et la vis 120. Le surmoulage peut alors être effectué.

En résumé, le procédé proposé permet d'assurer la coaxialité des différents éléments ainsi qu'un dimensionnement précis grâce à l'empilement 200 par emboîtement réalisé, lequel garantit également la compression des joints souples 222 à la mise en place sur la broche du moule. Le surmoulage confère une adhésion chimique qui crée une isolation électrique à la surface extérieure de la cavité 11, au niveau des zones 12 de fusion. L'ensemble des éléments constituant la cavité sont surmoulés dans la tête 10 de connecteur finale, facilitant l'industrialisation, cette dernière se réduisant à des opérations simples :
- moulage des manchons, demi-manchons, joints souples, et bouchons ;
- découpe au jet d'eau, électroérosion pour les contacts élastiques ;
- usinage sur commande numérique pour les cages des contacts électriques ; et
- surmoulage de l'ensemble dans le moule final de la tête de connecteur.

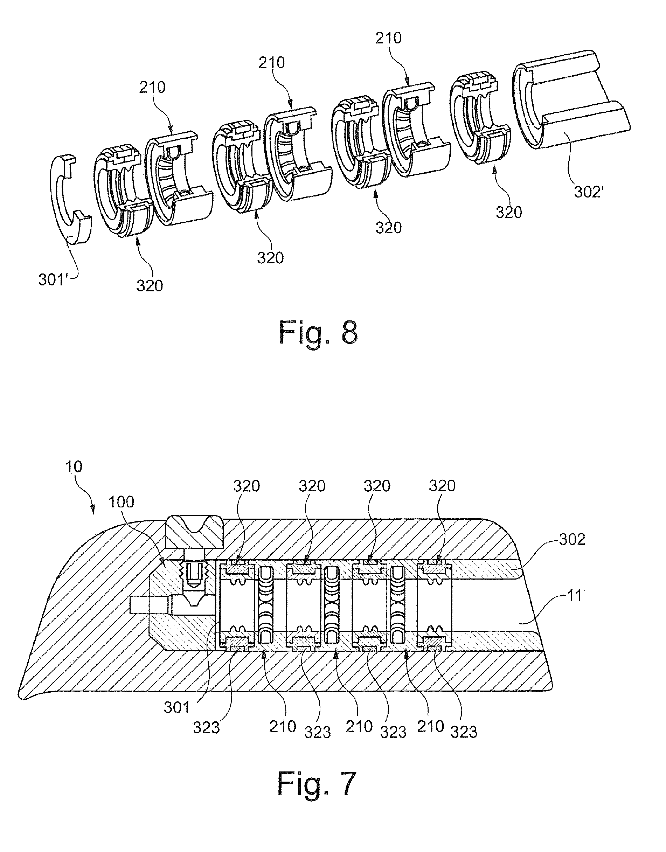

La Figure 7 propose une solution alternative à la tête de connecteur surmoulée des Figures 1 a et 1 b sous la forme d'une tête 10 de connecteur dans laquelle les éléments 210 de contact électrique et les éléments 320 d'isolement sont directement insérés par l'entrée d'une cavité 11 ouverte, préformée dans la tête 10.

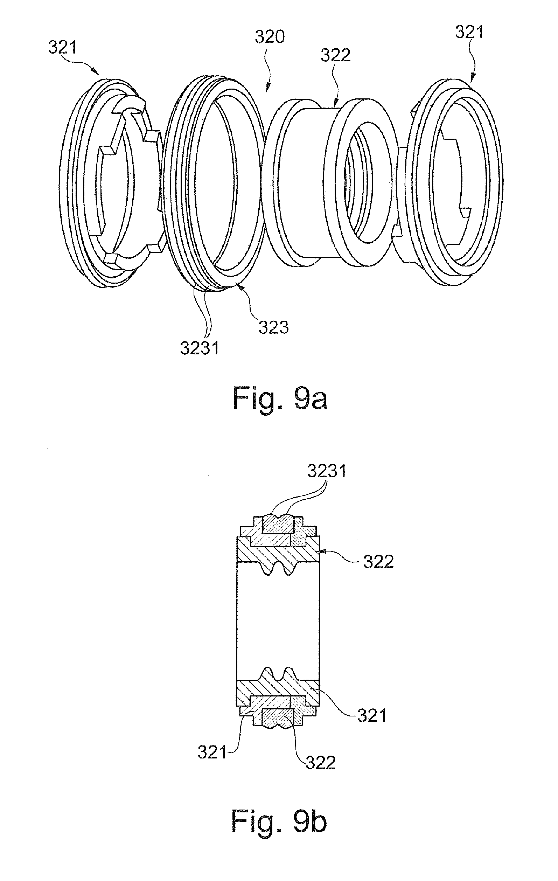

Dans cette variante, le manchon des éléments 320 d'isolement se compose, comme le montrent les Figures 9a et 9b, de deux demi-manchons 321 analogues à ceux représentés sur les Figures 4a, 4b et 5a, 5b. On notera cependant que les demi-manchons 321 portent sur leur face annulaire externe un deuxième joint souple 323 pour permettre de réaliser l'étanchéité entre les contacts électriques 210 et entre la cavité 11 et le milieu extérieur. L'isolation est alors obtenue par compression radiale des anneaux 3231 d'étanchéité des joints souples externes 323.

Après insertion dans la tête 10 de connecteur, les éléments annulaires 210, 320 présents dans la cavité sont mis en butée et maintenus en position entre les bouchons 301, 302 que l'on peut voir sur les Figures 7 et 8.

## Revendications

1. Un connecteur (10) pour un générateur de dispositif médical implan- table actif, comprenant une cavité (11) sensiblement cylindrique destinée à recevoir une sonde multipolaire du dispositif, la cavité comportant un empilement (200) d'éléments annulaires de contact électrique (210) séparés alternativement par des éléments annulaires d'isolement (220 ; 320) des éléments de contact électrique, dans lequel :
- les éléments d'isolement (220 ; 320) comprennent un manchon rigide annulaire (221) et un joint souple annulaire (222 ; 322) disposé, dans une région intérieure du manchon, contre une face annulaire interne (2211) du manchon ;
- le manchon et le joint comprennent des moyens respectifs (2212, 2213, 2222, 2223) d'immobilisation du joint par rapport au manchon en direction axiale, au moyen de profils conjugués définis respectivement sur une face annulaire interne (2211) du manchon et sur une face annulaire externe (2221) du joint ; et **caractérisé en ce que** :
- le joint s'étend axialement d'une face latérale du manchon jusqu'à l'autre dans ladite région intérieure du manchon ; et
- chacune des faces latérales (2225, 2226) du joint souple est en saillie par rapport à la face latérale correspondante du manchon.

2. Le connecteur de la revendication 1, dans lequel les profils conjugués sont définis par des épaulements (2212, 2213, 2222, 2223) ménagés en coopération sur la face annulaire interne du manchon et sur la face annulaire externe du joint.

3. Le connecteur de la revendication 1, dans lequel les profils conjugués comportent des gorges (2227) et des nervures (2219) ménagées en coopération sur la face annulaire externe du joint et sur la face annulaire interne du manchon.

4. Le connecteur de la revendication 1, comprenant en outre un deuxième joint souple annulaire (323), disposé sur la face annulaire externe du manchon.

5. Le connecteur de la revendication 1, dans lequel les éléments de contact électrique (210) et les manchons (221) des éléments d'isolement (220) comprennent des moyens d'assemblage par emboîtement (2215, 2216,2112,2113).

6. Le connecteur de la revendication 1, dans lequel le manchon est constitué de deux demi-manchons accolés.

7. Le connecteur de la revendication 1, constitué par surmoulage de l'empilement (200) d'éléments de contact électrique (210) et d'éléments d'isolement (220).

8. Le connecteur de la revendication 1, constitué par insertion des éléments de contact électrique (210) et des éléments d'isolement (320) à l'intérieur de la cavité (11).

9. Le connecteur de la revendication 1, dans lequel les éléments de contact électrique (210) comprennent une cage (211) logeant un contact élastique (212), le contact élastique comprenant une pièce métallique élastique présentant une section (2121) enroulée en forme de U pour constituer un cylindre ouvert, apte à réaliser une fonction de contact souple avec des bagues conductrices homologues de la fiche de connexion de la sonde.

10. Le connecteur de la revendication 9, dans lequel le contact élastique (212) est réalisé en une seule pièce à partir d'une feuille plate de métal élastique découpée de manière à y former des lamelles (2122), et pliée afin d'obtenir la forme en U (2121), puis le cylindre ouvert.

11. Le connecteur de la revendication 9, dans lequel la cage (211) comprend deux parois latérales (2114, 2115) formant butées axiales de positionnement pour les différents éléments annulaires d'isolement adjacents (220 ; 320) de l'empilement.

## Patentansprüche

1. Verbinder (10) für einen Generator einer aktiven implantierbaren medizinischen Vorrichtung, der einen im Wesentlichen zylindrischen Hohlraum (11) umfasst, der dazu bestimmt ist, eine Multipolsonde der Vorrichtung aufzunehmen, wobei der Hohlraum einen Stapel (200) aus ringförmigen Elementen (210) für elektrischen Kontakt enthält, die abwechselnd durch ringförmige Isolierelemente (220; 320) von Elementen mit elektrischen Kontakt getrennt sind, wobei:
- die Isolierelemente (220; 320) eine ringförmige starre Muffe (221) und eine ringförmige nachgiebige Verbindung (222; 322), die in einem inneren Bereich der Muffe gegenüber einer inneren ringförmigen Fläche (2211) der Muffe angeordnet ist, umfassen;
- die Muffe und die Verbindung jeweilige Mittel (2212, 2213, 2222, 2223) für die Blockierung der Verbindung in Bezug auf die Muffe in axialer Richtung mittels einander zugeordneter Profile, die auf einer inneren ringförmigen Fläche (2211) der Muffe bzw. einer äußeren ringförmigen Fläche (2221) der Verbindung definiert sind, umfassen; und **dadurch gekennzeichnet, dass**:
- die Verbindung in dem inneren Bereich der Muffe sich axial von einer Seitenfläche der Muffe bis zur anderen erstreckt; und
- jede der Seitenflächen (2225, 2226) der nachgiebigen Verbindung in Bezug von der entsprechenden Seitenfläche der Muffe vorsteht.

2. Verbinder nach Anspruch 1, wobei die einander zugeordneten Profile durch Schultern (2212, 2213, 2222, 2223) definiert sind, die zusammenwirkend auf der inneren ringförmigen Fläche der Muffe und auf der äußeren ringförmigen Fläche der Verbindung ausgebildet sind.

3. Verbinder nach Anspruch 1, wobei die einander zugeordneten Profile Nuten (2227) und Stege (2219) umfassen, die zusammenwirkend auf der äußeren ringförmigen Fläche der Verbindung und auf der inneren ringförmigen Fläche der Muffe ausgebildet sind.

4. Verbinder nach Anspruch 1, der außerdem eine zweite ringförmige nachgiebige Verbindung (323) umfasst, die auf der äußeren ringförmigen Fläche der Muffe angeordnet ist.

5. Verbinder nach Anspruch 1, wobei die Elemente (210) für elektrischen Kontakt und die Muffen (221) der Isolierelemente (220) Mittel (2215, 2216, 2112, 2113) für die Montage durch Einpassen umfassen.

6. Verbinder nach Anspruch 1, wobei die Muffe aus zwei zusammengeklebten Halbmuffen gebildet ist.

7. Verbinder nach Anspruch 1, der durch Übergießen des Stapels (200) von Elementen (210) für elektrischen Kontakt und von Isolierelementen (220) gebildet ist.

8. Verbinder nach Anspruch 1, der durch Einsetzen der Elemente (210) für elektrischen Kontakt und der Isolierelemente (320) in den Hohlraum (11) gebildet ist.

9. Verbinder nach Anspruch 1, wobei die Elemente (210) für elektrischen Kontakt einen Käfig (211) umfassen, der einen elastischen Kontakt (212) aufnimmt, wobei der elastische Kontakt ein elastisches Metallteil aufweist, das einen U-förmig aufgewickelten Abschnitt (2121) umfasst, um einen offenen Zylinder zu bilden, der dafür ausgelegt ist, eine Funktion für nachgiebigen Kontakt mit homologen leitenden Ringen des Verbindungssteckers der Sonde zu verwirklichen.

10. Verbinder nach Anspruch 9, wobei der elastische Kontakt (212) einteilig anhand eines ebenen elastischen Metallblechs verwirklicht ist, das so ausgeschnitten ist, dass Lamellen (2122) gebildet werden, und gebogen ist, um die U-Form (2121) und dann den offenen Zylinder zu erhalten.

11. Verbinder nach Anspruch 9, wobei der Käfig (211) zwei Seitenwände (2114, 2115) umfasst, die axiale Positionierungsanschläge für die verschiedenen benachbarten ringförmigen Isolierelemente (220; 320) des Stapels bilden.

## Claims

1. A connector (10) for a generator of an active implantable medical device, comprising a substantially cylindrical cavity (11) intended to receive a multipolar lead of the device, the cavity including a stack (200) of annular electrical contact elements (210) alternately separated by annular elements (220; 320) of isolation of the electrical contact elements, wherein:
- the isolation elements (220; 320) comprise an annular rigid sleeve (221) and an annular flexible gasket (222; 322) arranged, in an internal region of the sleeve, against an inner annular face (2211) of the sleeve;
- the sleeve and the gasket comprise respective means (2212, 2213, 2222, 2223) for immobilizing the gasket with respect to the sleeve in the axial direction, by means of conjugated profiles defined on an inner annular face (2211) of the sleeve and an outer annular face (2221) of the gasket, respectively; and **characterized in that**:
- the gasket extends axially from a lateral face of the sleeve to the other in said internal region of the sleeve; and
- each of the lateral faces (2225, 2226) of the flexible gasket protrudes with respect to the corresponding lateral face of the sleeve.

2. The connector of claim 1, wherein the conjugated profiles are defined by shoulders (2212, 2213, 2222, 2223) arranged in cooperation on the inner annular face of the sleeve and the outer annular face of the gasket.

3. The connector of claim 1, wherein the conjugated profiles include grooves (2227) and ribs (2219) arranged in cooperation on the outer annular face of the gasket and the inner annular face of the sleeve.

4. The connector of claim 1, further comprising a second annular flexible gasket (322), arranged on the outer annular face of the sleeve.

5. The connector of claim 1, wherein the electrical contact elements (210) and the sleeves (221) of the isolation elements (220) comprise interlocking connection means (2215, 2216, 2112, 2113).

6. The connector of claim 1, wherein the sleeve is consisted of two half-sleeves placed side-by-side.

7. The connector of claim 1, consisted by over-moulding of the stack (200) of electrical contact elements (210) and of isolation elements (220).

8. The connector of claim 1, consisted by insertion of the electrical contact elements (210) and the isolation elements (320) inside the cavity (11).

9. The connector of claim 1, wherein the electrical contact elements (210) comprise a cage (211) accommodating an elastic contact (212), the elastic contact comprising an elastic metal part having a cross-section (2121) rolled as a U-shape to constitute an open cylinder, adapted to fulfil a function of flexible contact with homologous conductive rings of the connection plug of the lead.

10. The connector of claim 9, wherein the electrical contact (212) is made as a single piece from a flat sheet of elastic metal cut so as to form therein platelets (2122), and folded so as to obtain the U-shape (2121), and the open cylinder.

11. The connector of claim 9, wherein the cage (211) comprises two lateral walls (2114, 2115) forming axial stops of positioning for the different adjacent annular isolation elements (220; 3220) of the stack.
